Europäisches Patentamt

(19) European Patent Office

Office européen des brevets

(11) Veröffentlichungsnummer: **0 006 207**
**A1**

(12) ## EUROPÄISCHE PATENTANMELDUNG

(21) Anmeldenummer: 79101876.5

(51) Int. Cl.³: **A 61 N 1/36, A 61 N 1/08**

(22) Anmeldetag: 11.06.79

(30) Priorität: 12.06.78 DE 2826054

(43) Veröffentlichungstag der Anmeldung: 09.01.80
Patentblatt 80/1

(84) Benannte Vertragsstaaten: **AT BE CH FR GB IT LU NL
SE**

(71) Anmelder: **Von Leitner, Enz-Rüdiger, Dr., Kiefernweg 8,
D-1000 Berlin 19 (DE)**

(72) Erfinder: **Von Leitner, Enz-Rüdiger, Dr., Kiefernweg 8,
D-1000 Berlin 19 (DE)**

(74) Vertreter: **Jander, Dieter, Dipl.-Ing. et al, Dipl.-Ing.
Dieter Jander Dr.-Ing. Manfred Böning Patentanwälte
Kurfürstendamm 66, D-1000 Berlin 15 (DE)**

(54) Implantierbarer Herzschrittmacher.

(57) Implantierbarer Herzschrittmacher mit einem Detektor, der
die elektrischen Herzaktionen, die den Stimulationsimpulsen
unmittelbar folgen, aufnimmt, und einem Schaltelement zur
späteren Erkennung von Fehlern bei der Impulsübertragung,
das in einem ersten Zustand verharrt, wenn die elektrische
Herzaktion eine bestimmte Zeit oder innerhalb eines bestimmten Zeitraumes nach dem Stimulationsimpuls kommt, und das in
einen zweiten Zustand gelangt, wenn die elektrische Herzaktion
später oder gar nicht kommt, wobei der erste oder zweite
Zustand bleibend erkennbar ist und von dem Schaltelement,
wenn dieses in seinen zweiten Zustand gelangt, die Stimulationsimpulsbreite und/oder die Stimulationsimpulsfrequenz
geändert, insbesondere vergrößert werden.

-1-

## Implantierbarer Herzschrittmacher

Die Erfingung bezieht sich auf einen implantierbaren Herzschrittmacher gemäss der im Oberbegriff des Anspruchs 1 erwähnten Art.

Bei einem bekannten Schrittmacher dieser Art (US-PS 4o 88 139) wird, wenn das Schaltelement in seinen zweiten Zustand gelangt, einerseits die Amplitude der Impulse vergrössert, womit man erreichen will, dass die Stimulationsimpulse wieder übergeleitet werden (s. Spalte 2, Zeilen 53 ff.), und andererseits werden zusätzlich zeitverschobene Impulse erzeugt, die die spätere Erkennung des Fehlverhaltens auf einem EKG-Diagramm ermöglichen (s. Spalte 3, Zeilen 7 ff.).

Der Erfindung liegt die Aufgabe zugrunde, die Sichtbarmachung eines Fehlverhaltens des Schrittmachers zu vereinfachen.

Diese Aufgabe wird erfindungsgemäss gelöst wie im Kennzeichen des Anspruchs 1 angegeben.

Die Änderung der Impulsbreite und der Impulsfrequenz ist schaltungstechnisch wesentlich einfacher durchführbar als die Schaffung neuer Impulse. Andererseits werden bei routinemässiger Überprüfung von Herzschrittmachern gerade diese beiden Grössen ohnedies herangezogen. Sie werden mit einfachen zigarettenschachtelgrossen Geräten erfasst. Zusätzliche Geräte und Arbeitsgänge entfallen somit. Mit den erwähnten Geräten lässt sich somit auch feststellen, ob der Schrittmacher durch ein früheres Fehlverhalten in seinen zweiten Zustand gesprungen ist. Wird die Impulsbreite vergrössert, so bringt das den weiteren Vorteil mit sich, dass pro Schrittmacherimpuls mehr Reizenergie an das Herz abgegeben wird. Das ist bei Erhöhung der Amplitude nicht der Fall, da der Übergangswiderstand zwischen Elektrode und Herzmuskel sehr gross ist. Die Verbreiterung der Impulse führt somit wieder zu Herzaktionen. Wird die Frequenz (bei wirksamer Stimulation) geändert, so ist man sogar in der Lage, ohne jegliches Gerät - allenfalls unter Zuhilfenahme einer Uhr - durch Messung des Pulses, z.B. am Handgelenk die Überprüfung vorzunehmen.

Zweckmässigerweise sollte die Umschaltung des Schaltelementes blockiert sein, wenn der Herzschrittmacher festfrequent arbeitet. Das ist z.B. dann der Fall, wenn der Herzschrittmacher Störfeldern ausgesetzt ist.

9. Herschrittmacher nach einem oder mehreren der Ansprüche 1 bis 8, dadurch gekennzeichnet, dass das Umspringen in den zweiten Zustand nach einem oder mehrmaligem Ausbleiben (unmittelbar oder nicht unmittelbar hintereinander) der zeitgerechten elektrischen Herzaktion(en) vorzugsweise innerhalb eines vorgegebenen Zeitraumes erfolgt.

10. Herzschrittmacher nach einem oder mehreren der Ansprüche 1 bis 9, dadurch gekennzeichnet, dass der Zustand des Schaltelementes durch ein optisches und/oder akustisches Signal in einem exsternen Kontrollgerät angezeigt wird.

0006207

- 1 -

Patentansprüche :

1. Implantierbarer Herzschrittmacher mit einem Detektor, der die elektrischen Herzaktionen, die den Stimulationsimpulsen unmittelbar folgen, aufnimmt, und einem Schaltelement, das in einem ersten Zustand verharrt, wenn die elektrische Herzaktion eine bestimmte Zeit oder innerhalb eines bestimmten Zeitraumes nach dem Stimulationsimpuls kommt, und das in einen zweiten Zustand gelangt, wenn die elektrische Herzaktion später oder gar nicht kommt, wobei der erste oder zweite Zustand bleibend erkennbar ist, dadurch gekennzeichnet, dass von dem Schaltelement, wenn dieses in seinen zweiten Zustand gelangt, die Stimulationsimpulsbreite und/oder die Stimulationsimpulsfrequenz geändert, insbesondere vergrössert werden.

2. Herzschrittmacher nach Anspruch 1, dadurch gekennzeichnet, dass die Stimulationsfrequenz insbesondere von 7o auf ca. 9o bis 1oo Impulse pro Minute geändert wird.

3. Herzschrittmacher nach Anspruch 1 oder 2, dadurch gekennzeichnet, dass die Stimulationsfrequenz bei Auflegen eines Prüfmagneten insbesondere von 7o auf ca. 9o bis 1oo pro Minute geändert wird.

4. Herzschrittmacher nach einem oder mehreren der Ansprüche 1 bis 3, dadurch gekennzeichnet, dass die Impulsbreite verdoppelt wird.

5. Herzschrittmacher nach einem oder mehreren der Ansprüche 1 bis 4, dadurch gekennzeichnet, dass ein Kondensator vorgesehen ist, der jedesmal, durch den Stimulationsimpuls ausgelöst, ge- oder entladen wird und dessen Ladungszustand das Schaltelement betätigt, sofern nicht vorher die elektrische Herzaktion aufgetreten ist.

6. Herzschrittmacher nach einem oder mehreren der Ansprüche 1 bis 5, dadurch gekennzeichnet, dass das Schaltelement durch Eingriff von aussen wieder in seinen ersten Zustand zurückgebracht werden kann.

7. Herzschrittmacher nach einem oder mehreren der Ansprüche 1 bis 6, dadurch gekennzeichnet, dass bei Abnehmen des zum Feststellen der Frequenz dienenden Prüfmagneten das Schaltelement in seinen ersten Zustand zurückgelangt.

8. Herzschrittmacher nach einem oder mehreren der Ansprüche 1 bis 7, dadurch gekennzeichnet, dass die Umschaltung des Schaltelementes blockiert ist, wenn der Schrittmacher festfrequent arbeitet.

| | Europäisches Patentamt | **EUROPÄISCHER RECHERCHENBERICHT** | 0006207 Nummer der Anmeldung EP 79 10 1876 |

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | <u>US - A - 3 920 024</u> (VITATRON)<br>* Spalte 2, Zeilen 52-59; Spalte 6, Zeilen 20-31; Spalte 14, Zeilen 15-36 *<br>-- | 1,6,8 |
| | <u>US - A - 3 949 758</u> (MEDTRONIC)<br>* Spalte 4, Zeilen 31-33; Spalte 3, Zeilen 38-41; Spalte 8, Zeilen 1-36; Spalte 5, Zeilen 55-63 *<br>-- | 1,5 |
| D | <u>US - A - 4 088 139</u> (MEDALERT)<br>* Spalte 3, Zeilen 31-44; Spalte 8, Zeile 38 - Spalte 9, Zeile 60; Spalte 10, Zeilen 3-7 *<br>-- | 1,4,6 7,9 |
| | <u>US - A - 3 759 265</u> (AMERICAN OP-<u>TICAL</u>)<br>* Spalte 2, Zeilen 53-66; Spalte 6, Zeilen 9-19 *<br>-- | 1 |
| | <u>DE - A - 2 146 965</u> (DEVICES)<br>* Seite 3, Abschnitte 1 und 2 *<br>-- | 1,2 |
| | <u>FR - A - 2 159 450</u> (MEDTRONIC)<br>* Seite 7, Zeile 32 - Seite 8, Zeile 28 *<br>--<br>./. | 5 |

**KLASSIFIKATION DER ANMELDUNG (Int.Cl.²)**

A 61 N 1/36
A 61 N 1/08

**RECHERCHIERTE SACHGEBIETE (Int Cl.²)**

A 61 N 1/36
A 61 N 1/08

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 14-09-1979 | SIMON |

EPA form 1503.1   06.78

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | FR - A - 2 269 922 (DAINI SEIKOSHA) <br> * Seite 1, Zeilen 7-12; Ansprüche 15 und 16 * <br> -- | 10 | |
| | US - A - 3 782 367 (HOFFMANN-LA ROCHE) <br> * Spalte 10, Zeilen 62-68; Spalte 11, Zeilen 45-47 * <br> -- | 10 | RECHERCHIERTE SACHGEBIETE (Int. Cl.²) |
| PE | FR - A - 2 374 025 (CORDIS) <br> * Seite 2, Zeilen 10-26; Seite 8, Zeile 32 - Seite 9, Zeile 34 * | 1 | |
| PE | & NL - A - 77 13831 <br> -- | | |
| P | US - A - 4 102 345 (AMERICAN OPTICAL) <br> * Spalte 6, Zeilen 5-43 * <br> -- | 3 | |
| A | DE - A - 1 937 136 (LIECHTI) <br> * Seite 13, letzter Abschnitt; Seite 14, erster Abschnitt; Seite 16, erster Abschnitt * <br> ---- | 1,6,7 | |